(19) Europäisches Patentamt
European Patent Office
Office européen des brevets

(11) EP 0 989 127 A1

(12) **EUROPEAN PATENT APPLICATION**

(43) Date of publication:
**29.03.2000 Bulletin 2000/13**

(51) Int Cl.⁷: **C07D 311/94**, A61K 31/35, A01N 43/16

(21) Application number: **99307231.3**

(22) Date of filing: **13.09.1999**

(84) Designated Contracting States:
**AT BE CH CY DE DK ES FI FR GB GR IE IT LI LU MC NL PT SE**
Designated Extension States:
**AL LT LV MK RO SI**

(30) Priority: **14.09.1998 WOPCT/IB98/01412**

(71) Applicant: **PFIZER INC.**
**New York, N.Y. 10017 (US)**

(72) Inventors:
- **Kato, Yoshinao**
**Chita-gun, Aichi-ken 470-2393 (JP)**
- **Parkinson, Tanya**
**Sandwich, Kent CT13 9NJ (GB)**
- **Watanabe, Shuzo**
**Chita-gun, Aichi-ken 470-2393 (JP)**

(74) Representative:
**Simpson, Alison Elizabeth Fraser et al**
**Urquhart-Dykes & Lord,**
**91 Wimpole Street**
**London W1M 8AH (GB)**

(54) **An antifungal lactone compound**

(57) This invention provides processes for producing a terpenoid lactone compound. which comprises cultivating *Codinaea talbotii* FERM BP-6321 and then isolating the terpenoid lactone compound from the fermentation broth. The compound produced by these processes include the terpenoid lactone compound of the following formula:

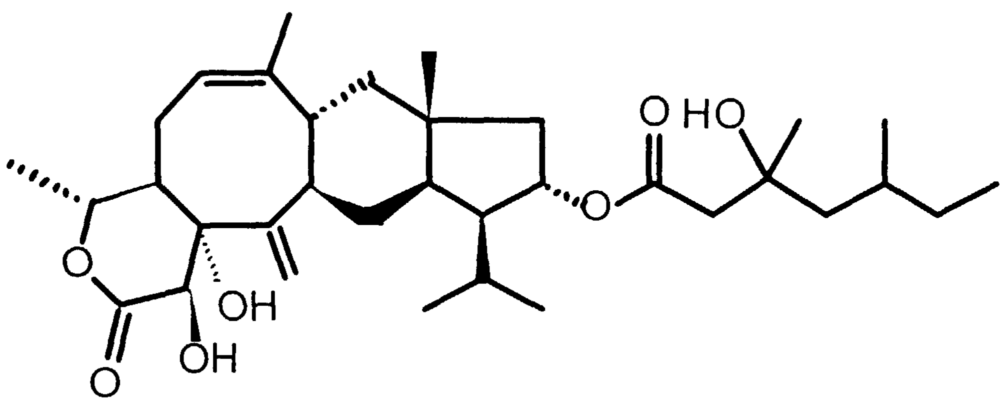

The present invention also relates to a pharmaceutical composition comprising the same, which is useful in the treatment of fungal infection or the like.

EP 0 989 127 A1

## Description

### Technical Field

This invention relates to an antifungal agent comprising a terpenoid lactone compound, particularly the terpenoid compound produced by fermentation of an fungus *Codinaea talbotii* which has been deposited as FERM BP-6321. This invention also relates to processes for producing the terpenoid lactone compound, and a pharmaceutical composition comprising the same, which is useful in the treatment of fungal infection in animals including humans.

### Background Art

A terpenoid lactone compound of formula (I) has been reported as a potent inhibitor of glycosylphosphatidylinositol (GPI)-synthesis (C. Sutterlin et al., The EMBO Journal, 1997, 16, 6374). The terpenoid lactone compound blocks GPI-synthesis in yeast and mammalian cells but not in protozoa, suggesting that the GPI-biosynthetic machinery be species specific. However, there is no description about the compound as a antifungal agent in the published paper.

### Brief Disclosure of the Invention

This invention provides an antifungal agent comprising a compound of the following formula or its pharmaceutically acceptable salts:

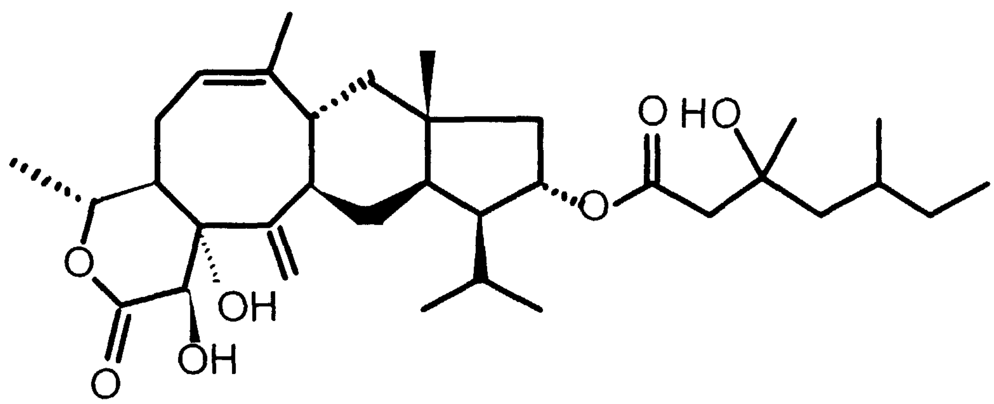

(I).

This invention also provides a culture of *Codinaea talbotii* FERM-6321 which is capable of producing a compound of formula (I). The compound of formula (I) exhibits antifungal activity with low cytotoxicity to mammalian cells. Thereofore, this invention also provides a pharmaceutical composition for use in the treatment of fungal infection, comprising a pharmaceutically acceptable carrier and an efective antifungal amount of a compound of formula (I).

This invention also provides a process for preparing the compound of formula (I), comprising aerobically fermenting the culture FERM-6321, or a mutant or a recombinant form thereof and recovering said compound by separation from the fermentation medium.

Further, this invention is directed to a method for antifungal treatment of a subject in need of such treatment which comprises administering to said subject an antifungal amount of a compound of formaula (I) or its pharmaceutically acceptable salts.

### Detailed Description of the Intention

The microorganism used in this invention is a strain of *Codinaea talbotii* which was obtained from the American Type Culture Collection. It was deposited on April 8, 1998 under the accession number FERM BP-6321 to National Institute of Bioscience and Human-Technology, Agency of Industrial Science and Technology (located at 1-3 Higashi 1-chome, Tsukuba, Ibaraki 305, Japan) under the Budapest Treaty. The taxonomical properties of this strain have been reported by Watanabe, T. (Trans. Mycol. Soc. Japan, 16, 149 - 182, 1975). describing that this strain is hyphomycete *Codinaea talbotii.* This fungus formed slimy spore heads apically or rarely laterally on the simple conidiophores. Conidiophores pale brown, 3-5-separate. erect from the sustaining hyphae, often broader in the intermediate portion than the basal portion. 68.1-389.2 μm long, 2.9-4.7 μm in the maximum width, never branched. with apical and lateral openings with colarettes; lateral phialidal openings about μm broad. 1.2 μm deep. Hyphae pale yellow. Conidia hyaline,

cylindrical, slightly curved in one direction, 1-celled, 9.5-13.4 x 2.9-3.4 μm.

In this invention, a mutant or recombinant form of *Codinaca talbotii* FERM BP-6321 having the ability to produce the terpenoid lactone compound of formula (I), can be also used. The mutant or recombinant form may be obtained by spontaneous mutation, artificial mutation with ultraviolet radiation, or treatment with mutagen such as N-methyl-N'-nitro-N-nitrosoguanidine or ethyl methanesulfonate, or a cell technology method such as cell fusion, gene manipulation or the like, according to well-known methods.

According to the present invention. the terpenoid lactone compound of formula (I) may be produced by aerobic fermentation of *Codinaea talbotii* FERM BP-6321, or a mutant or recombinant form thereof, under conditions similar to those generally employed to produce bioactive compounds by fermentation.

FERM BP-6321, or a mutant or recombinant form thereof, is usually fermented on solid medium with an insoluble material and aqueous nutrient media. The amount of the insoluble material may be in the range of 10 to 50% (w/v). Suitable insoluble materials useful for fermentation include sand, cracked stone, wood chip and whole broken grains, such as wheat bran, oatmeal, cracked corn, millet, etc. In this invention, cultivation of FERM BP-6321 to produce the antifungal terpenoid lactone compound is preferably carried out using such insoluble materials and aqueous nutrient media at a temperature of 20 to 35 °C for 3 to 20 days. preferablly 14 to 21 days. The pH of the medium may be adjusted in the range from 4.0 to 9.0, preferably from 5.0 to 7.5.

Nutrient media useful for fermentation include a source of assimilable carbon such as sugars, starches and glycerol; and a source of organic nitrogen such as casein, enzymatic digest of casein, soybean meal, cotton seed meal, peanut meal, wheat gluten, soy flour, meat extract and fish meal. A source of growth substances such as mineral salts, sodium chloride and calcium carbonate; and trace elements such as iron. magnesium, copper, zinc, cobalt and manganese may also be utilized with advantageous results. If excessive foaming is encountered during fermentation, antifoam agents such as polypropylene glycols or silicons may be added to the fermentation medium.

Aeration of the medium in fermenters for submerged growth is maintained at 3 to 200%, preferably at 50 to 150% volumes of sterile air per volume of the medium per minute. The rate of agitation depends on the type of agitator employed. A shake flask is usually run at 150 to 250 rpm whereas a fermenter is usually run at 300 to 2.000 rpm. Aseptic conditions must, of course, be maintained through the transfer of the organism and throughout its growth.

The terpenoid lactone compound thus produced may be isolated by standard techniques such as extraction and various chromatographic techniques.

The production of the terpenoid lactone compound produced by the process of this invention may be measured by the standard in vitro protocol described below:

## Determination of Antifungal activity by Minimum Inhibitory Concentration method (M.I.C.)

The in vitro evaluation of the antifungal activity of the compound of formula (I) can be performed by determining the minimum inhibitory concentration, which is the concentration of the test compound in a suitable medium at which grouwth of the particular microorganism fails to occur. A frozen stock of *Candida albicans* cells is thawed out at room temperature. The suspension of the *Candida albicans* cells (5 μl) is inoculated into 20 ml of Saubraund Dextrose Broth (SAB) (Difco) in a 125 ml flask, and is incubated at 30°C in a shaker incubator for 18 hours at 200 rpm. One ml of the cells is inoculated into 30 ml of SAB in a 250 ml flask, and is incubated at 30°C in a shaker incubator for 3 to 4 hours at 200 rpm to reach the log phase of growth. The optical density (OD at 650 nm) of the cell culture is diluted to an OD of 0.0025 in a Beckman spectrophotometer (DU-640B). Seventy five μl of the diluted *Candida albicans* cells are transferred to each well of the asssay microplate with 25 μl of test sample. The plate is incubated at 30°C for 16 to 20 hours in a humidified incubator. The plate is read on a microplate reader (BioRad model 3550-UV) at 650 nm using a 7 second pre-mixing. Antifungal activity is calculated by the formula:

$$\text{Inhibition (\%)} = \{1- \frac{\text{(A650 Sample - A650 Blank)}}{\text{(A650 Control - A650 Blank)}}\} \times 100$$

## Determination of Cytotoxicity by Crystal Violet Staining

The in vitro evaluation for the cytotoxicity of the compound of formula (I) can be performed by monitoring the growth inhibition of culture cells. HeLa cells are cultivatcd in Eagle's Minimum Essential Medium (Nissui) supplemented with 10 % (v/v) fetal calf serum, 100 units/ml penicillin, 100 μg ml streptomycin and 2 mM L-glutamine in a humidified CO2 (5%) incubator at 37 °C. HepG2 cells are cultivated in Dulbecco's Modified Eagle's Medium (Gibco BRL) supplemented with 10 % (v/v) fetal calf serum, 40 μg/ml gentamicin and 2 mM L-glutamine. For complete detachment, confluent cells are rinsed with Dulbecco's Phosphate- Buffered Saline (PBS) once and allowed to detach with 0.05 % (w/v) trypsin - 0.53 mM EDTA for 5 min at 37 °C. The cell suspension is centrifuged at 450 x g for 5 min at 4 °C. For the regular passage, each cell pellet is suspended in the above medium and split to new flasks. For the assay, the cell

pellet is suspended in the above medium to be 5.6 x $10^4$ cells/ml. Serial dilution of the compound of formula (I) is made in methanol or 10 % (v/v) DMSO, and 20 μl of samples are dispensed into 96-well tissue culture plates. Cell suspension (180 μl, 1 x $10^4$ cells) is added to each well and incubated for 72 h. Crystal violet is dissolved in methanol to be 0.4 % (w/v) solution and filtered through a paper filter. After 72 h, culture medium is removed by decantation and the cells are washed once with PBS. Fifty, μl of crystal violet solution is added to each well and the plates are left for 30 min at room temperature. Crystal violet solution is removed by decantation and the plates are washed 10 times with tapped water. After drying for 1 h at room temperature, 50 μl of 50 % (v/v) methanol is dispensed and the plates are swirled until crystal violet is completely solubilized (over 10 min). The plates are read on a microplate reader (Bio-Rad model 3550) at 490 nm. Cytotoxicity is calculated by the following formula:

$$\text{Cytotoxicity (\%)} = \{1- (\text{A490 Sample} - \text{A490 Blank})/(\text{A490 Control} - \text{A490 Blank})\} \times 100$$

## Administration

The antifungal agent of this invention comprising theterpenoid lactone compound of formula (I) is useful in the treatment of fungal infection or the like. The antifungal terpenoid lactone compound may be administered alone or in combination with pharmaceutically acceptable carriers, in either single or multiple doses. Suitable pharmaceutical carriers include inert solid diluents or fillers, sterile aqueous solution and various organic solvents. The pharmaceutical compositions formed by combining the terpenoid lactone compound and the pharmaceutically acceptable carriers are then readily administered in a variety of dosage forms such as tablets, powders, lozenges. syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally. lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredients therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of the terpenoid lactone compound in sesame or peanut oil, aqueous propylene glycol, or in sterile aqueous solution may be employed. Such aqueous solutions should be suitablely buffered if necessary and the liquid diluent first rendered isotonic with sufficient saline or glucose. These particular aqueous solutions are especially suitable for intravenous, intramuscular, subcutaneous and intraperitioneal administration. In this connection, the sterile aqueous media employed are all readily available by standard techniques known to those skilled in the art.

Additionally, the antifungal terpenoid lactone compound may be administered topically when treating conditions of the skin and this may be done by way of creams, jellies, gels, pastes, and ointments, in accordance with standard pharmaceutical practice.

In general, the antifungal terpenoid lactone compound is present in the above dosage forms at concentration levels ranging 5 to 70 % by weight, preferably 10 to 50% by weight.

In general, a therapeutically effective daily dose for the active compound will range from 0.01 to 100 mg/kg, generally from about 1 to about 5 mg/kg As is generally known, the effective dosage for the active compound depends on the intended route of administration and other factors such as age and weight of the patient. as generally known to a physician. The dosage also depends on the illnes to be treated.

## Examples

The present invention is illustrated by the following examples. However, it should be understood that the invention is not limited to the specific details of these examples. Spectral data were obtained by the following instruments: NMR, JEOL Lambda 270 and FAB-MS, JEOL JMS-700. All NMR spectra were measured in $CD_3OD$ unless otherwise indicated and peak positions are expressed in parts per million (ppm) based on the reference of $CD_3OD$ peak at 3.3 ppm for $^1H$ NMR and 49.8 ppm for $^{13}C$ NMR. The peak shapes are denoted as follows: s (singlet), d (doublet), t (triplet), q (quartet), m (multiplet) and br (broad). FAB-MS spectra were measured using glycerol-matrix.

## Example One

## Fermentation of *Codinaea talbotii* FERM BP-5778

One hundred (100) ml of Medium-1 (potato dextrose broth 2.4%, yeast extract 0.5% and agar 0.1%) in 500-ml flask was inoculated with a vegetative cell suspension from a slant culture of *Codinaea talbotii* FERM BP-6321. The flask was shaken at 26°C for 4 days on a rotary shaker with 7-cm throw at 210 rpm, to obtain a seed culture.
The seed culture was used to inoculate into five 500-ml flasks containing 100 ml of Medium-2 (glucose 1%, glycerol 6.6%, NZ Amine Type A 0.5%, ammonium sulfate 0.2%, defatted soybean meal 0.5%, tomato paste 0.5%, sodium citrate 0.2% and pH 7.0) and 30 g buckwheat. Incubation was carried out at 26°C for 18 days.

## Extraction and isolation

The fermentation broth (500 ml) was filtered after the addition of 500 ml of ethanol. The filtrate was concentrated to aqueous solution (300 ml), which was then extracted 3 times with each of 300 ml of n-hexane. The extract was evaporated to afford an oily residue. The oily residue (440 mg) dissolved in methanol (1 ml) was applied to a YMC-pack ODS AM-343 column (20 x 250 mm. Yamamura) and eluted with methanol - water (85:15) for 30 min at a flow rate of 8 ml/min. The detection was made by UV absorbance at 220 nm. The eluted peak showing activity was collected to yield the terpenoid lactone (40.7 mg).
The terpenoid lactone compound of formula (I) was isolated in a substantially pure from the fermentation mixture.

## HPLC analysis

Analytical HPLC of the terpenoid lactone compound was performed using a YMC-pack ODS AM-312 column (6.0 x 150 mm, Yamamura) and eluted with methanol - water (85:15) at a flow rate of 0.9 ml min. The retention time of terpenoid lactone was 7.8 min.

## Characterization

The spectral data of the terpenoid lactone compound were as follows: colorless powdery molecular formula $C_{34}H_{54}O_7$; LRFAB-MS m/z 575 $[M+H]^+$; HRFAB-MS m/z 575.3839; $^1$H NMR d 5.28 (dt, *J* = 7.8 and 3.2 Hz, 1H), 5.14 (br s, 1H), 5.10 (br t, J= 7.8 Hz, 1H), 4.68 (br s, 1H), 4,29 (dq, J= 10.5 and 6.2 Hz, 1H), 4.18 (s, 1H), 3.15 (m, 1H), 3.11 (m, 1H), 2.48 (d, *J* = 14.3 Hz, 1H), 2.42 (d, *J* = 14.3 Hz, 1H), 2.30~2.05 (m, 3H), 2.04~1.86 (m, 4H), 1.85~1.68 (m, 2H), 1.64 (s, 3H), 1.63~1.52 (m, 2H), 1.41 (d *J* = 6.2 Hz, 3H), 1.5~1.3 (m, 3H), 1.27 (s, 3H), 1.30~1.06 (m, 3H), 1.00 (s, 3H), 0.97 (d,*J* = 5.9 Hz. 3H), 0.95 (d, *J* = 6.2 Hz, 3H), 0.90 (d, *J* = 4.9 Hz, 3H), 0.88 (t, *J* = 7.6 Hz, 3H); $^{13}$C NMR δ 175.44 (s), 172.90 (s), 152.31 (s), 143.99 (s), 118.51 (d), 111.18 (t), 81.00 (d), 78.01 (d), 77.57 (s), 75.77 (d), 72.89 (s), 54.87 (d), 52.15 (d), 51.53 (d), 49.21 (t), 48.58 (t), 47.63 (t), 47.33 (t), 47.12 (d), 44.83 (d), 43.47 (s), 35.51 (t), 32.33 (t), 31.48 (d), 31.00 (d), 27.91 (q), 26.55 (q), 24.45 (q), 22.54 (t), 22.32 (q), 21.71 (q), 21.02 (q), 19.88 (q), 11.70 (q).
Based on various spectroscopic techniques such as UV spectrophotometry, NMR and mass spectrometries. the terpenoid lactone compound of formula (I) was identified as YW3548 that was reported by Sutterlin et al (The EMBO J. 1997, 16, 6374).

## Antifungal activity and cytotoxicity

The inhibitory activitiy of the terpenoid lactone compound (I) on the growth of *Candida albicans* and *Aspergillus fumigatus* was determined by the above-shown M.I.C. under the condition described herein above. The MIC values against *Candida albicans* and *Aspergillus fumigatus* were each < 0.19 µg/ml. The compound also inhibited the growth of *Candida albicans* and *Aspergillus fumigatus* with MFC values of < 0.19 and >25 µg/ml (84% killed at 25 µg/ml), respectively. Cytotoxicities of the compound against HepG2 and HeLa cella were tested according to the method described above. Cytotoxicities to the both type of the cells were not observed at 5 µg/ml.

| Antifungal activity (µg/ml) | |
|---|---|
| Tested fungi | MIC |
| *Candida albicans* | < 0.19 |
| *Aspergillus fumigatus* | < 0.19 |

(continued)

| Antifungal activity (µg/ml) | | |
|---|---|---|
| | | |
| Concentration (µg/ml) | | Cytotoxicity (% inhibition) |
| *HepG2* | 5 | 16 |
| *HeLa* | 5 | 15 |

**Claims**

1. An antifungal agent comprising a compound of the following formula or pharmaceutically acceptable salts thereof:

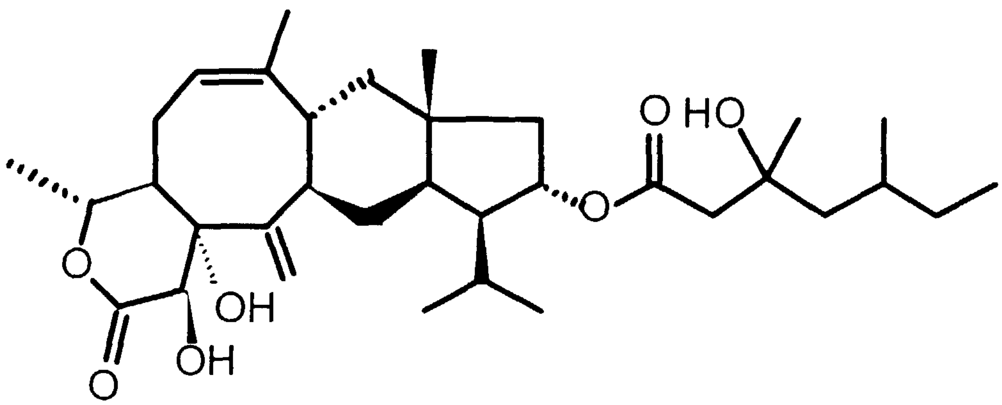

(I).

2. A pharmaceutical composition for use in the treatment of fungal infection, comprising a pharmaceutically acceptable carrier and an effective antifungal amount of a compound of the formula (I) according to claim 1.

3. A culture of *Codinaea talbotii* FERM-6321 which is capable of producing a compound according to claim 1.

4. A process for preparing the compound according to claim 1, comprising aerobically fermenting the culture FERM-6321, or a mutant or a recombinant form thereof and recovering said compound by separation from the fermentation medium.

5. A method for antifungal treatment of a subject in need of such treatment which comprises administering to said subject an antifungal amount of a compound according to claim 1 or a pharmaceutically acceptable salts thereof.

European Patent Office

# EUROPEAN SEARCH REPORT

Application Number
EP 99 30 7231

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A,P | CHEMICAL ABSTRACTS, vol. 130, no. 3, 18 January 1999 (1999-01-18) Columbus, Ohio, US; abstract no. 22650, WANG, YING ET AL: "Conformation and relative configuration of a very potent glycosylphosphatidylinositol-anchoring inhibitor with an unusual tricarbocyclic sesterterpenoid.delta.-lactone skeleton from the fungus Paecilomyces inflatus" XP002122608 * abstract * & HELV. CHIM. ACTA (1998), 81(11), 2031-2042 , --- | 1-5 | C07D311/94<br>A61K31/35<br>A01N43/16 |
| A,P | CHEMICAL ABSTRACTS, vol. 129, no. 12, 21 September 1998 (1998-09-21) Columbus, Ohio, US; abstract no. 145898, SUTTERLIN, CHRISTINE ET AL: "Saccharomyces cerevisiae GPI10, the functional homolog of human PIG-B, is required for glycosylphosphatidylinositol-anchor synthesis" XP002122609 * abstract * & BIOCHEM. J. (1998), 332(1), 153-159 , --- -/-- | 1-5 | |
| | | | TECHNICAL FIELDS SEARCHED (Int.Cl.7) |
| | | | C07D<br>A61K<br>A01N |

The present search report has been drawn up for all claims

| Place of search | Date of completion of the search | Examiner |
|---|---|---|
| BERLIN | 16 November 1999 | Frelon, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 99 30 7231

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | CHEMICAL ABSTRACTS, vol. 128, no. 8, 23 February 1998 (1998-02-23) Columbus, Ohio, US; abstract no. 86268, SUTTERLIN, CHRISTINE ET AL: "Identification of a species-specific inhibitor of glycosylphosphatidylinositol synthesis" XP002122610 * abstract * & EMBO J. (1997), 16(21), 6374-6383 , ----- | 1-5 | |
| | | | TECHNICAL FIELDS SEARCHED (Int.Cl.7) |

The present search report has been drawn up for all claims

| Place of search | Date of completion of the search | Examiner |
|---|---|---|
| BERLIN | 16 November 1999 | Frelon, D |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)